# FASCICULE DE BREVET EUROPEEN

(11) **EP 1 861 083 B1**
(45) Date de publication et mention de la délivrance du brevet: **31.01.2018**
(21) Numéro de dépôt: 06726096.8
(22) Date de dépôt: 15.03.2006
(51) Int. Cl.: A61K 31/20, A61K 31/7048, A61P 17/00, A61P 17/08, A61P 17/10

(54) **COMPOSITION À BASE D'UNE AVERMECTINE ET D'ACIDE AZÉLAÏQUE NOTAMMENT POUR LE TRAITEMENT DE LA ROSACÉE**
ZUBEREITUNG AUF BASIS EINES AVERMECTINS UND AZEALINSÄURE, INSBESONDERE ZUR BEHANDLUNG VON ROSAZEA
COMPOSITION BASED ON AN AVERMECTIN AND AZELAIC ACID IN PARTICULAR FOR TREATING ROSACEA

(30) Priorité: 17.03.2005 FR 0502645
(43) Date de publication de la demande: 05.12.2007
(73) Titulaire: Galderma S.A., 6330 Cham (CH)
(72) Inventeur: KAOUKHOV, Alexandre, F-06160 Juan les Pins (FR); PERNIN, Colette, F-06200 Nice (FR)
(74) Mandataire: Tezier Herman, Béatrice
(86) Numéro de dépôt international: PCT/FR2006/000570
(87) Numéro de publication internationale: WO 2006/097628

(56) Documents cités:
- WO-A-2004/022046
- FR-A- 2 854 074
- LOO W J ET AL: "INVERMECTIN CREAM IN ROSACEA: COMPARISON WITH METRONIDAZOLE GEL" BRITISH JOURNAL OF DERMATOLOGY, XX, XX, vol. 151, juillet 2004 (2004-07), pages 61,ABSTRNOP-92, XP009056313 ISSN: 0007-0963
- FORTON F ET AL: "Demodicosis and rosacea: Epidemiology and significance in daily dermatologic practice" JOURNAL OF THE AMERICAN ACADEMY OF DERMATOLOGY, C.V. MOSBY, ST. LOUIS, MO, US, vol. 52, no. 1, janvier 2005 (2005-01), pages 74-87, XP004717805 ISSN: 0190-9622

## Description

La présente invention se rapporte à une composition pharmaceutique, et notamment dermatologique, destinée au traitement d'affections de la peau, notamment au traitement de la rosacée (anciennement dénommée acné rosacée). En particulier, l'invention se rapporte à une composition pharmaceutique, notamment dermatologique, comprenant, dans un milieu physiologiquement acceptable, au moins un composé de la famille des avermectines, l'acide azélaïque et un gélifiant choisi parmi le mélange Sodium acryloyldiméthyltaurate copolymer / isohexadecane / polysorbate 80 et/ou le mélange polyacrylamide / isoparaffine C13-14 / laureth-7.

Elle concerne en outre l'utilisation d'une telle composition pour la fabrication d'un médicament destiné au traitement d'affections de la peau, en particulier la rosacée.

La rosacée est une dermatose inflammatoire chronique affectant principalement la partie médiane du visage et les paupières de certains adultes. Elle est caractérisée par un érythème télangiectasique, une sécheresse de la peau, des papules et des pustules.

Classiquement, la rosacée se développe chez les adultes entre l'âge de 30 et 50 ans; elle atteint plus fréquemment les femmes bien que l'affection soit généralement plus sévère chez les hommes.

Malgré son ancien nom, l'acné rosacée n'est pas une affection du follicule pilosébacé comme l'acné juvénile mais une affection primitivement vasculaire dont le stade inflammatoire est dépourvu de kystes et de comédons caractéristiques de l'acné vulgaire.

L'étiologie de la rosacée est encore mal comprise, bien que de nombreuses théories aient été élaborées. La thèse la plus commune est basée sur la présence caractéristique du parasite *Demodex folliculorum* chez les patients atteints de rosacée. Cet organisme est absent dans l'acné vulgaire. D'autres facteurs ont été décrits, comme pouvant contribuer au développement de la rosacée, tels que les facteurs hormonaux et notamment endocrines, les facteurs climatiques, immunologiques et bactériens par la présence de *Helicobacter pylori,* une bactérie associée aux désordres gastro-intestinaux.

La rosacée évolue en quatre stades sur plusieurs années par poussées aggravées par les variations de température, l'alcool, les épices, l'exposition solaire, les émotions. Les différents stades de la maladie sont les suivants :
Stade 1: Stade des épisodes d'érythème. Les patients ont des poussées d'érythrose due à la dilatation brutale des artérioles du visage qui prend alors un aspect congestif, rouge. Ces poussées sont provoquées par les émotions, les repas, les changements de température.
Stade 2: Stade de la couperose, c'est à dire de l'érythème permanent avec télangiectasies. Certains patients présentent également des oedèmes au niveau des joues et du front.
Stade 3: Stade inflammatoire avec apparition de papules et pustules inflammatoires mais sans atteinte du follicule sébacé et donc avec une absence de kystes et de comédons.
Stade 4: Stade du rhinophyma. Cette phase tardive touche essentiellement les hommes. Les patients présentent un nez volumineux, rouge, bosselé avec une hyperplasie sébacée et un remaniement fibreux du tissu conjonctif.

Classiquement, la rosacée est traitée oralement ou topiquement par des antibiotiques tels que les tétracyclines, l'érythromycine, la clindamycine, le métronidazole, mais aussi par la vitamine A, l'acide salicylique, des agents antifongiques, des stéroïdes, des anti-infectieux tels que le peroxyde de benzoyle, par l'isotrétinoïne ou encore par l'acide azélaïque.

L'acide azélaïque (ou acide 1,7-heptanedicarboxylique) est connu dans l'art antérieur pour ses propriétés anti-acnéiques et kératolytiques. L'acide azélaïque possède une activité antibactérienne sur *P. acnes* et sur *S*. *epidermidis.* Il inhibe la prolifération des kératinocytes, diminue le taux d'acide gras libres dans les sécrétions sébacées et possède également une activité anti-inflammatoire.

La demande de brevet WO2004/022046 décrit une méthode de traitement de la rosacée par application topique d'une composition à base d'acide azélaïque et de métronidazole.

La demande de brevet FR 2 854 074 A décrit l'utilisation de l'ivermectine dans le traitement de désordres dermatologiques tels que la rosacée, l'acné vulgaire, et la dermatite séborrhéique.

L'étude publiée par Loo et al. (British Journal of Dermatology, 2004, 151, suppl.68, page 61) compare l'efficacité de l'ivermectine et du metronidazole dans le traitement de la rosacée.

Le brevet US 5,952,372 décrit également une méthode de traitement de la rosacée utilisant de manière orale ou topique l'ivermectine afin de réduire et éliminer le parasite *Demodex folliculorum* présent sur la peau des patients.

L'ivermectine appartient à la famille des avermectines, un groupe de lactones macrocycliques produit par la bactérie Streptomyces avermitilis (Reynolds JEF (Ed) (1993) Martindale. The extra pharmacopoeia. 29th Edition. Pharmaceutical Press, London). Les avermectines incluent notamment l'ivermectine, l'invermectine, l'avermectine, l'abamectine, la doramectine, l'eprinomectine et la sélamectine.

L'ivermectine est connu dans l'art antérieur pour ses propriétés antiparasitaires et anthelminthiques. L'activité antiparasitaire serait due à l'ouverture d'un canal chlore au niveau de la membrane des neurones du parasite sous l'effet d'une libération accrue du neuromédiateur GABA (gamma-aminobutyric acid), induisant une paralysie neuromusculaire pouvant conduire à la mort de certains parasites. L'ivermectine interagit également avec d'autres canaux chlore, notamment ceux dépendant du neuromédiateur GABA (gamma-aminobutyric acid). Il est déjà décrit chez l'homme dans le traitement de l'onchocercose à Onchocerca volvulus, de la strongyloïdose (anguillulose) gastro-intestinale (produit Stromectol®), de la gale sarcoptique humaine (Meinking TL et al., N Engl J Med 1995 Jul 6;333(1):26-30 The treatment of scabies with ivermectin) ainsi que dans le traitement de la microfilarémie diagnostiquée ou suspectée chez les sujets atteints de la filariose lymphatique due à Wuchereria bancrofti. Le brevet US 6,133,310 décrit l'utilisation de l'ivermectine dans le traitement de la rosacée afin de réduire et éliminer le parasite *Demodex folliculorum* présent sur la peau des patients.

Toutefois, ces traitements présentent des inconvénients tels que des phénomènes d'irritation et d'intolérance notamment lorsqu'ils sont utilisés de manière prolongée. De plus, ces traitements sont uniquement suppressifs et non curatifs, en agissant notamment sur les poussées pustuleuses développées lors du stade inflammatoire.

Considérant la nature chronique de la rosacée, le traitement idéal nécessite un usage prolongé et ceci d'une manière sûre et efficace. Tenant compte de ce qui précède, il existe donc un besoin de réaliser une composition qui montre une efficacité améliorée dans le traitement de la rosacée et qui ne présente pas les effets secondaires décrits dans l'art antérieur. Il existe notamment un besoin de réaliser une composition conférant une plus grande tolérance des principes actifs, tout en diminuant leurs effets secondaires.

C'est pourquoi la présente description décrit une composition comprenant l'association d'au moins un composé de la famille des avermectines et de l'acide azélaïque, utile dans le traitement de la rosacée.

L'invention a pour objets respectifs une composition pharmaceutique et l'utilisation d'une telle composition dans la prévention et/ou le traitement d'une affection de la peau telles que définies dans les revendications.

L'invention a donc pour objet une composition pharmaceutique, notamment dermatologique, comprenant, dans un milieu physiologiquement acceptable, au moins un composé de la famille des avermectines et l'acide azélaïque, et ses sels, et un gélifiant choisi parmi le mélange Sodium acryloyldiméthyltaurate copolymer / isohexadecane / polysorbate 80 et/ou le mélange polyacrylamide / isoparaffine C13-14 / laureth-7. Par milieu physiologiquement acceptable, on entend tout milieu compatible avec la peau, les muqueuses et/ou les phanères.

L'acide azélaïque selon l'invention peut être utilisé en tant que tel, ou bien sous la forme d'un sel avec une base pharmaceutiquement acceptable, ou encore selon la présente description sous forme d'un dérivé. Par dérivés, on entend des composés qui se distinguent de l'acide azélaïque par substitution, addition ou suppression d'un ou plusieurs groupements chimiques et qui présentent sensiblement la même activité.

L'invention a donc pour objet une composition pharmaceutique, notamment dermatologique, comprenant, dans un milieu physiologiquement acceptable, au moins un composé de la famille des avermectines, et au moins un composé choisi parmi l'acide azélaïque et ses sels, et un gélifiant choisi parmi le mélange Sodium acryloyldiméthyltaurate copolymer / isohexadecane / polysorbate 80 et/ou le mélange polyacrylamide / isoparaffine C13-14 / laureth-7.

L'invention a préférentiellement pour objet une composition pharmaceutique, notamment dermatologique, comprenant, dans un milieu physiologiquement acceptable, au moins de l'ivermectine et l'acide azélaïque, et un gélifiant choisi parmi le mélange Sodium acryloyldiméthyltaurate copolymer / isohexadecane / polysorbate 80 et/ou le mélange polyacrylamide / isoparaffine C13-14 / laureth-7.

L'invention a également pour objet l'utilisation d'une telle composition pour la fabrication d'un médicament destiné à la prévention et/ou au traitement d'une affection de la peau.

L'invention et les avantages qui en découlent seront mieux compris à la lecture de la description de modes de réalisation non limitatifs qui suivent.

Les composés de la famille des avermectines utilisables selon la présente invention incluent notamment l'invermectine, l'ivermectine, l'avermectine, l'abamectine, la doramectine, l'eprinomectine et la sélamectine. De manière préférentielle, le composé de la famille des avermectines est l'ivermectine.

Dans les compositions selon l'invention, ledit composé de la famille des avermectines est présent à des concentrations comprises entre 0,001 et 10 % en poids, par rapport au poids total de la composition, de préférence entre 0,01 et 5 % en poids.

Dans les compositions selon l'invention, l'acide azélaïque et ses sels sont présents à des concentrations comprises entre 0,01 et 40 % en poids par rapport au poids total de la composition, de préférence entre 1 et 20 %.

Dans l'ensemble du présent texte, à moins qu'il ne soit spécifié autrement, il est entendu que lorsque des intervalles de concentrations sont donnés, ils incluent les bornes supérieures et inférieures dudit intervalle.

Avantageusement, les compositions de l'invention comprennent, outre au moins un composé de la famille des avermectines et l'acide azélaïque, au moins un autre agent actif thérapeutique susceptible d'augmenter l'efficacité du traitement. A titre d'exemples non limitatifs de tels agents, on peut citer des antibiotiques, des agents antibactériens (comme le métronidazole), des agents antiviraux, des antiparasitaires, des agents antifongiques, des anesthésiques, des analgésiques, des antiallergiques, des rétinoïdes, des anti-radicaux libres, des antiprurigineux, des kératolytiques, des antiséborrhéiques, des anti-histaminiques, des sulfures, des produits immunosuppresseurs ou antiprolifératifs ou un mélange de ceux-ci.

Les compositions selon l'invention peuvent comprendre en outre tout adjuvant habituellement utilisé dans le domaine dermatologique, compatible avec ledit composé de la famille des avermectines et l'acide azélaïque. On peut citer notamment des agents chélatants, des antioxydants, des filtres solaires, des conservateurs, des charges, des électrolytes, des humectants, des colorants, des bases ou des acides usuels, minéraux ou organiques, des parfums, des huiles essentielles, des actifs cosmétiques, des hydratants, des vitamines, des acides gras essentiels, des sphingolipides, des composés autobronzants, des agents apaisants et protecteurs de la peau, des agents propénétrants ou un mélange de ceux-ci. Ces additifs, ainsi que leur concentration doivent être tels qu'ils ne nuisent pas aux propriétés avantageuses du mélange selon l'invention. Ces additifs peuvent être présents dans la composition à raison de 0 à 20 % en poids par rapport au poids total de la composition, de préférence de 1 à 10 % en poids.

Comme conservateurs, on peut citer à titre d'exemple, le chlorure de benzalkonium, le phénoxyéthanol, l'alcool benzylique, la diazolidinylurée, les parabens ou leurs mélanges.

Comme agents humectants, on peut citer en particulier, la glycérine et le sorbitol.

Comme agents chélatants, on peut citer à titre d'exemple, l'acide éthylènediaminetétracétique (EDTA), ainsi que ses dérivés ou ses sels, la dihydroxyethylglycine, l'acide citrique, l'acide tartrique ou leurs mélanges.

Comme agents propénétrants, on peut citer en particulier, le propylène glycol, le dipropylène glycol, le propylène glycol dipélargonate, le lauroglycol et l'ethoxydiglycol.

Les compositions selon l'invention sont utiles pour le traitement et/ou la prévention de la rosacée.

Selon un premier mode de mise en oeuvre de l'invention, l'utilisation de la composition est destinée à la fabrication d'un médicament pour le traitement et/ou la prévention d'une affection de la peau et de préférence pour le traitement et/ou la prévention de la rosacée, de l'acné vulgaire et/ou de la dermatite séborrhéique et de manière particulièrement préférée pour le traitement de la rosacée.

L'invention se rapporte encore à l'utilisation d'au moins un composé de la famille des avermectines pour la préparation d'une composition pharmaceutique, notamment dermatologique, destinée à la prévention et/ou au traitement d'une affection de la peau, le composé de la famille des avermectines étant associé dans ladite composition avec l'acide azélaïque. Dans cette utilisation, la composition est comme définie précédemment.

La composition selon l'invention est de préférence d'application topique.
La composition selon l'invention est une composition pharmaceutique, et notamment dermatologique, qui peut se présenter sous toutes les formes galéniques classiquement utilisées pour une application topique et notamment sous forme de gels aqueux, de solutions aqueuses ou hydroalcooliques. Elle peut aussi, par ajout d'une phase grasse ou huileuse, se présenter sous forme de dispersions du type lotion ou sérum, d'émulsions de consistance liquide ou semi-liquide du type lait obtenues par dispersion d'une phase grasse dans une phase aqueuse (H/E) ou inversement (E/H), ou de suspensions ou émulsions de consistance molle semi-liquide ou solide du type crème ou gel ou pommade ou encore d'émulsions multiples (E/H/E ou H/E/H), de micro-émulsions, de micro-capsules, de micro-particules ou de dispersions vésiculaires de type ionique et/ou non ionique, ou des dispersions cire/phase aqueuse. Ces compositions sont préparées selon les méthodes usuelles.

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine dermatologique. L'émulsionnant et le co-émulsionnant sont généralement présents dans la composition, en une proportion allant de 0,3 à 30% en poids, et de préférence de 0,5 à 20% en poids par rapport au poids total de la composition. L'émulsion peut en outre contenir des vésicules lipidiques.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclomethicone) et les huiles fluorées (perfluoropolyethers). On peut aussi utiliser comme matières grasses des alcools gras tels que l'alcool cétylique, des acides gras, des cires et des gommes en particulier les gommes de silicone.

Comme émulsionnants et co-émulsionnants utilisables dans l'invention, on peut citer par exemple les esters d'acide gras et de polyéthylène glycol tels que le stéarate de PEG-100, le stéarate de PEG-50 et le stéarate de pEG-40; les esters d'acide gras et de polyol tels que le stéarate de glycéryle, le tristéarate de sorbitane et les stéarates de sorbitane oxyéthylénés disponibles sous les dénominations commerciales Tween 20 ou Tween 60, par exemple; et leurs mélanges. A titre d'exemples non limitatifs de gélifiants utiles selon la présente description, on peut citer la famille des polyacrylamides tels que le mélange Sodium acryloyldiméthyltaurate copolymer / isohexadecane / polysorbate 80 vendu sous le nom Simulgel™ 600 par la société Seppic™, le mélange polyacrylamide / isoparaffine C13-14 / laureth-7 comme, par exemple, celui vendu sous le nom de Sepigel 305™ par la société Seppic™, la famille des polymères acryliques couplés à des chaînes hydrophobes tel que le PEG-150 / decyl / SMDI copolymère vendu sous le nom de Aculyn 44™ (polycondensat comprenant au moins comme éléments, un poléthylèneglycol à 150 ou 180 moles d'oxyde d'éthylène, de l'alcool décylique et du méthylène bis(4-cyclohexylisocyanate) (SMDI), à 35 % en poids dans un mélange de propylèneglycol (39 %) et d'eau (26 %)), la famille des amidons modifiés tels que l'amidon de pomme de terre modifié vendu sous le nom de Structure Solanace™ ou bien leurs mélanges.

Les gélifiants selon l'invention sont le mélange Sodium acryloyldiméthyltaurate copolymer / isohexadecane / polysorbate 80 et/ ou le mélange polyacrylamide / isoparaffine C13-14 / laureth-7. Le gélifiant tel que décrit ci-dessus peut être utilisé à une concentration allant de 0,1 à 15 % et, de préférence de 0,5 à 5 %.

## Revendications

1. Composition pharmaceutique, notamment dermatologique, **caractérisée en ce qu'**elle comprend, dans un milieu physiologiquement acceptable, au moins un composé de la famille des avermectines, au moins un composé choisi parmi l'acide azélaïque et ses sels, et un gélifiant choisi parmi le mélange Sodium acryloyldiméthyltaurate copolymer / isohexadecane / polysorbate 80 et/ou le mélange polyacrylamide / isoparaffine C13-14 / laureth-7.

2. Composition selon la revendication 1, **caractérisée en ce que** le composé de la famille des avermectines est choisi parmi l'invermectine, l'ivermectine, l'avermectine, l'abamectine, la doramectine, l'eprinomectine et la sélamectine.

3. Composition selon la revendication 1 ou 2, **caractérisée en ce que** le composé de la famille des avermectines est l'ivermectine.

4. Composition selon l'une quelconque des revendications 1 à 3, **caractérisée en ce que** le composé de la famille des avermectines représente entre 0,001 et 10 % en poids, par rapport au poids total de la composition, de préférence entre 0,01 et 5 % en poids.

5. Composition selon l'une quelconque des revendications 1 à 4, **caractérisée en ce que** la concentration d'acide azélaïque et/ou de ses sels est comprise entre 0,01 et 40 % en poids, par rapport au poids total de la composition, de préférence entre 1 et 20 %.

6. Composition selon l'une quelconque des revendications 1 à 5, **caractérisée en ce qu'**elle est d'application topique.

7. Composition selon l'une quelconque des revendications 1 à 6, **caractérisée en ce qu'**elle contient en outre au moins un agent actif choisi dans le groupe comprenant les antibiotiques, les agents antibactériens, les antiviraux, les antiparasitaires, les antifongiques, les anesthésiques, les analgésiques, les antiallergiques, les rétinoïdes, les anti-radicaux libres, les antiprurigineux, les kératolytiques, les antiséborrhéiques, les anti-histaminiques, les sulfures, les produits immunosuppresseurs ou antiprolifératifs.

8. Composition selon l'une quelconque des revendications 1 à 7 **caractérisée en ce qu'**elle contient en outre au moins un additif choisi dans le groupe comprenant les agents chélatants, les antioxydants, les filtres solaires, les conservateurs, les charges, les électrolytes, les humectants, les colorants, les bases ou les acides usuels, minéraux ou organiques, les parfums, les huiles essentielles, les actifs cosmétiques, les hydratants, les vitamines, les acides gras essentiels, les sphingolipides, les composés autobronzants, les agents apaisants et protecteurs de la peau, les agents propénétrants, ou un mélange de ceux-ci.

9. Utilisation d'une composition selon l'une quelconque des revendications 1 à 8 pour la fabrication d'un médicament destiné à la prévention et/ou au traitement d'une affection de la peau.

10. Utilisation selon la revendication 9, **caractérisée en ce que** le médicament est destiné au traitement et/ou à la prévention de la rosacée, de l'acné vulgaire et/ou de la dermatite séborrhéique.

11. Utilisation selon la revendication 9 ou 10, **caractérisée en ce que** le médicament est destiné au traitement et/ou à la prévention de la rosacée.

12. Composition selon l'une quelconque des revendications 1 à 8, pour son utilisation dans la prévention et/ou le traitement d'une affection de la peau.

13. Composition pour son utilisation selon la revendication 12, dans le traitement et/ou la prévention de la rosacée, de l'acné vulgaire et/ou de la dermatite séborrhéique.

14. Composition pour son utilisation selon la revendication 13, dans le traitement et/ou la prévention de la rosacée.

## Patentansprüche

1. Pharmazeutische Zusammensetzung, insbesondere dermatologische Zusammensetzung, **dadurch gekennzeichnet, dass** sie in einem physiologisch verträglichen Medium wenigstens eine Verbindung aus der Familie der Avermectine, wenigstens eine Verbindung, ausgewählt aus Azelainsäure und ihren Salzen, und einen Gelbildner, ausgewählt aus dem Gemisch aus Natriumacryloyldimethyltaurat-Copolymer / Isohexadecan / Polysorbat 80 und/oder dem Gemisch aus Polyacrylamid / C13-14-Isoparaffin / Laureth-7, umfasst.

2. Zusammensetzung gemäß Anspruch 1, **dadurch gekennzeichnet, dass** die Verbindung aus der Familie der Avermectine aus Invermectin, Ivermectin, Avermectin, Abamectin, Doramectin, Eprinomectin und Selamectin ausgewählt ist.

3. Zusammensetzung gemäß Anspruch 1 oder 2, **dadurch gekennzeichnet, dass** die Verbindung aus der Familie der Avermectine Ivermectin ist.

4. Zusammensetzung gemäß einem der Ansprüche 1 bis 3, **dadurch gekennzeichnet, dass** die Verbindung aus der Familie der Avermectine zwischen 0,001 und 10 Gew.-%, vorzugsweise zwischen 0,01 und 5 Gew.-% bezogen auf das Gesamtgewicht der Zusammensetzung ausmacht.

5. Zusammensetzung gemäß einem der Ansprüche 1 bis 4, **dadurch gekennzeichnet, dass** die Konzentration der Azelainsäure und/oder ihrer Salze zwischen 0,01 und 40 Gew.-%, vorzugsweise zwischen 1 und 20 Gew.-% bezogen auf das Gesamtgewicht der Zusammensetzung beträgt.

6. Zusammensetzung gemäß einem der Ansprüche 1 bis 5, **dadurch gekennzeichnet, dass** sie topisch appliziert wird.

7. Zusammensetzung gemäß einem der Ansprüche 1 bis 6, **dadurch gekennzeichnet, dass** sie außerdem wenigstens ein aktives Agens enthält, ausgewählt aus der Gruppe, umfassend Antibiotika, antibakterielle Agenzien, antivirale Agenzien, Antiparasitika, Fungizide, Anästhetika, Analgetika, Antiallergika, Retinoide, Radikalfänger, Antipruriginosa, Keratolytika, Antiseborrhoika, Antihistaminika, Sulfide, immunsupprimierende oder antiproliferative Mittel.

8. Zusammensetzung gemäß einem der Ansprüche 1 bis 7, **dadurch gekennzeichnet, dass** sie außerdem wenigstens einen Zusatzstoff enthält, ausgewählt aus der Gruppe, umfassend Chelatbildner, Antioxidantien, Sonnenschutzfilter, Konservierungsstoffe, Füllstoffe, Elektrolyte, Feuchthaltemittel, Farbstoffe, übliche organische oder anorganische Säuren oder Basen, Duftstoffe, essentielle Öle, kosmetische Wirkstoffe, hydratisierende Mittel, Vitamine, essentielle Fettsäuren, Sphingolipide, selbstbräunende Verbindungen, Hautberuhigungs- und Hautschutzmittel, penetrationsfördernde Mittel oder ein Gemisch davon.

9. Verwendung einer Zusammensetzung gemäß einem der Ansprüche 1 bis 8 für die Herstellung eines Arzneimittels zur Prävention und/oder Behandlung einer Hauterkrankung.

10. Verwendung gemäß Anspruch 9, **dadurch gekennzeichnet, dass** das Arzneimittel für die Behandlung und/oder Prävention der Rosacea, der Acne vulgaris und/oder der Dermatitis seborrhoica bestimmt ist.

11. Verwendung gemäß Anspruch 9 oder 10, **dadurch gekennzeichnet, dass** das Arzneimittel für die Behandlung und/oder Prävention der Rosacea bestimmt ist.

12. Zusammensetzung gemäß einem der Ansprüche 1 bis 8 für die Verwendung in der Prävention und/oder Behandlung einer Hauterkrankung.

13. Zusammensetzung für die Verwendung gemäß Anspruch 12 in der Behandlung und/oder Prävention der Rosacea, der Acne vulgaris und/oder der Dermatitis seborrhoica.

14. Zusammensetzung für die Verwendung gemäß Anspruch 13 in der Behandlung und/oder Prävention der Rosacea.

## Claims

1. A pharmaceutical composition, especially a dermatological composition, **characterized in that** it comprises, into a physiologically acceptable medium, at least one compound of the avermectin family, at least one compound selected from among azelaic acid and salts thereof, and a gelling agent selected from among the sodium acryloyldimethyltaurate copolymer/isohexadecane/polysorbate 80 mixture and/or the polyacrylamide/C13-14 isoparaffin/Laureth-7 mixture.

2. The composition according to claim 1, **characterized in that** the compound of the avermectin family is selected from among invermectin, ivermectin, avermectin, abamectin, doramectin, eprinomectin and selamectin.

3. The composition according to claim 1 or 2, **characterized in that** the compound of the avermectin family is ivermectin.

4. The composition according to any one of claims 1 to 3, **characterized in that** the compound of the avermectin family ranges from 0.001 % to 10% by weight, relative to the total weight of the composition, preferably from 0.01% to 5% by weight.

5. The composition according to any one of claims 1 to 4, **characterized in that** the concentration of azelaic acid and/or salts thereof ranges from 0.01% and 40% by weight, relative to the total weight of the composition, preferably from 1% to 20%.

6. The composition according to any one of claims 1 to 5, **characterized in that** it is for topical application.

7. The composition according to any one of claims 1 to 6, **characterized in that** it further comprises at least one active agent selected from the group comprising antibiotics, anti-bacterial agents, anti-viral agents, anti-parasitic agents, anti-fungal agents, anaesthetics, analgesics, anti-allergic agents, retinoids, free-radical scavengers, anti-pruritic agents, keratolytic agents, anti-seborrhoeic agents, anti-histamines, sulfides, and immunosuppressant or anti-proliferative products.

8. The composition according to any one of claims 1 to 7, **characterized in that** it further comprises at least one additive selected from the group comprising chelating agents, antioxidants, sunscreens, preservatives, fillers, electrolytes, humectants, dyes, mineral or organic acids or bases, fragrances, essential oils, cosmetic active agents, moisturizers, vitamins, essential fatty acids, sphingolipids, self-tanning compounds, skin-soothing and protecting agents, pro-penetrating agents, or a mixture thereof.

9. Use of a composition according to any one of claims 1 to 8, for manufacturing a medicament for preventing and/or treating of a skin affliction.

10. Use according to claim 9, **characterized in that** the medicament is for treating and/or preventing rosacea, common acne and/or seborrhoeic dermatitis.

11. Use according to claim 9 or 10, **characterized in that** the medicament is for treating and/or preventing rosacea.

12. The composition according to any one of claims 1 to 8, for use in preventing and/or treating a skin affliction.

13. The composition according to claim 12, for use in treating and/or preventing rosacea, common acne and/or seborrhoeic dermatitis.

14. The composition according to claim 13, for use in treating and/or preventing rosacea.
